Europäisches Patentamt

European Patent Office

Office européen des brevets

(19)

(11) Publication number: **0 140 478**
**A2**

# (12) EUROPEAN PATENT APPLICATION

(21) Application number: **84304836.4**

(22) Date of filing: **16.07.84**

(51) Int. Cl.⁴: **A 61 F 5/44**

(30) Priority: **18.07.83 US 515012**

(43) Date of publication of application:
**08.05.85 Bulletin 85/19**

(84) Designated Contracting States:
**AT BE CH DE FR GB IT LI LU NL SE**

(71) Applicant: **Hanson, James Phillip**
**8516 West G Avenue**
**Kalamazoo Michigan 49009(US)**

(72) Inventor: **Hanson, James Phillip**
**8516 West G Avenue**
**Kalamazoo Michigan 49009(US)**

(74) Representative: **Robinson, Anthony John Metcalf et al,**
**Kilburn & Strode 30 John Street**
**London, WC1N 2DD(GB)**

(54) Disposable incontinence product.

(57) A disposable diaper (10; 110) having improved liquid receiving and retaining capabilities, includes a waterproof barrier formed as a flattened bag (12; 112) and having a single opening (14; 114) located to be adjacent the perineal area of the body of the wearer. Two layers of filler material are positioned within the barrier bag. The upper layer adjacent the opening is a wicking material (16; 116), while the lower layer is a superabsorbent material (18; 118). In a male version of the diaper, a pouch (136) is formed over the opening into which the wearer's penis is inserted to direct voided urine through the bag opening.

EP 0 140 478 A2

./...

Croydon Printing Company Ltd.

FIG. 3

# DISPOSABLE INCONTINENCE PRODUCT

The present invention relates to incontinence products, and more particularly to disposable incontinence products.

A wide variety of incontinence products have been developed for the incontinent adult. These products are typically disposable and include a water impervious barrier sheet and an absorbent material positioned thereon. In use, the waterproof barrier supports the absorbent material against the wearer's body, such that the absorbent material is positioned to absorb voided urine.

In one particularly effective product, the water impervious layer is formed as a flattened bag having a single opening proximate the perineal area of the wearer. An absorbent material is located within the bag and accessible through the opening. Examples of this type of diaper are described in US-A-4 285 342, US-A-2 201 255, and US-A-2 532 029.

However, these diapers are not without their drawbacks. Most importantly, the absorbent material within the bag is typically incapable of adequately rapidly absorbing a high flow rate of voided urine. Consequently, the voided urine will often overflow the diaper, resulting in wetting of the wearer's clothes.

This problem is overcome by one aspect of the present invention, according to which an incontinence product comprises a waterproof barrier formed into a flattened, substantially closed bag including upper and lower surfaces, the upper surface defining an aperture to be located proximate the perineal area of a wearer; a wicking material within said bag proximate said opening; and an absorbent material proximate said wicking material, whereby when the wearer voids, urine passes through said bag aperture and is rapidly transported by said wicking material to said absorbent material. With this construction, when the wearer voids into the product, the wicking material rapidly accepts and conveys the large volume of urine away from the opening to a large portion of the absorbent material to eliminate overflow problems. Therefore, even with relatively large volumes and flow rates of voided urine, the diaper is capable of transporting and absorbing the volume sufficiently rapidly to prevent wetting of the wearer's clothes.

A further problem with the diapers described in the aforementioned specifications is that they are best suited for women where the diaper bag opening can be located proximate the urethra to properly receive urine. These diapers are a problem for males wherein the position of the urethra outlet shifts with activity of the wearer and often is not directed into the bag opening at the time of voiding. If urine is voided when the urethra is not directed toward the opening, the urine simply flows off the water impervious bag and wets the wearer's clothes.

Although male drip absorbing devices are known, they typically do not include sufficient volume of absorbent to receive a full volume of voided urine and they are awkward to use and expose the user to undesirable urine contact against large areas of the skin. Example of these drip-absorbing products are shown in US-A-2 873 740 and US-A-2 445 220.

These problems are overcome by a second aspect of the present invention according to which an incontinence product for males comprises a flattened bag fabricated of a liquid-impervious material, the bag including first and second opposite surfaces, one of the said surfaces defining an opening positioned to be located proximate the perineal area of a wearer when the product is worn; and filler material means within the bag for receiving urine, at least one of the bag and the filler material means including pouch means adjacent said opening for receiving the wearer's penis to ensure that voided urine is directed through said opening into said filler material means.

The invention may be carried into practice in various ways but two incontinence diapers embodying the invention will now be described by way of example with reference to the accompanying drawings, in which:

Figure 1 is a perspective view of the first incontinence diaper with the water impervious bag partially separated to show the bag interior;

Figure 2 is a sectional view taken along plane II-II in Figure 1 and additionally showing a facing material overlying the bag;

Figure 3 is a perspective view of the second diaper with the water impervious bag partially separated to show the bag interior;

Figure 4 is a sectional view taken along plane IV-IV in Figure 3 and additionally showing a facing material overlying the bag; and

Figure 5 is a sectional view taken along plane V-V in Figure 3.

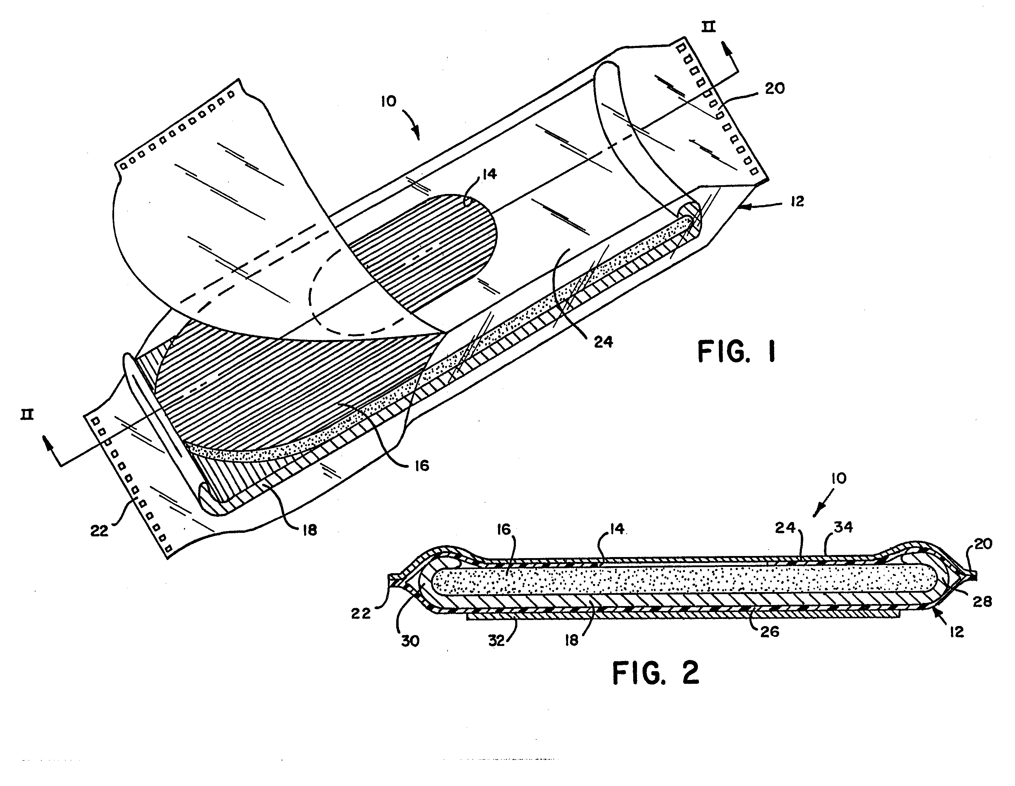

The incontinence diaper 10 illustrated in Figures 1 and 2 basically includes a liquid impervious bag 12, having a single perineal opening 14, and two layers 16 and 18 of material located within the bag. Layer 18 is an absorbent material and preferably a superabsorbent material to absorb and retain urine voided into the diaper 10 through the opening 14. Layer 16 is a wicking material positioned between the opening 14 and the superabsorbent layer 18 to rapidly transport liquid from the opening 14 to a wide area over the absorbent material layer 18 to improve the urine reception rate of the diaper.

Turning more specifically to the construction of the diaper 10, the bag or liquid barrier 12 is fabricated of any suitable liquid-impermeable material of tubular stock. In the preferred embodiment, either polypropylene or polyethylene having a weight of approximately 12 grams per square metre is used. Of course, other suitably impervious materials can be substituted for those of the preferred embodiment. The barrier 12 has a tube-shaped configuration and is heat-sealed or adhesively bonded using conventional techniques at each of its opposite ends 20 and 22 to form a substantially watertight bag. In the preferred embodiment, the barrier 12 is approximately 203 mm (8 inches) long, 63.5 mm (2.5 inches) wide, and 19 mm (.75 inch) deep. The opening 14 is generally elongated and formed in the upper side 24 of the bag 12. In the preferred embodiment, the opening 14 is located generally centrally, both longitudinally and transversely, on the upper side 24 and is approximately 63.5 mm (2.5 inches) long and 25.4 mm (1 inch) wide.

The wicking material 16 may be any wicking material or combination of wicking materials generally available to those in the art. "Wicking material" has a meaning generally recognized in the art as a material selected for its relatively rapid liquid transportation characteristics, rather than liquid absorption characteristics. A preferred material has a high percentage of open area to provide "reservoir" space to aid in accepting high flow rates of urine. The preferred material should be resilient and not collapse when wetted. In the preferred embodiment, the material 16 comprises a liquid-permeable fibrous web manufactured under the trademark CORWEB by British Vita and having a density of approximately 0.03 $gm/cm^3$. The wicking material 16 is approximately 16.5 mm (6.5 inches) long by 63.5 mm (2.5 inches) wide by 12.7 mm (.50 inch) deep.

The superabsorbent 18 is an absorbent material selected for its liquid absorption and retention capabilities. In the preferred embodiment, the absorbent 18 is three layers of the superabsorbent sold by Dow under the trademark DWAL. The dimensions of layer 18 are approximately 203 mm (8.0 inches) long by 63.5 mm (2.5 inches) wide by 6.3 mm (.25 inch) deep. The opposite ends 28 and 30 of layer 18 are folded about wicking material 16 to improve absorption of liquid from the wicking material. Although layers 16 and 18 have been described as two separate materials, it will be appreciated by those having ordinary skill in the art that layers 16 and 18 could be integrated into a single material having layers with the described characteristics, and that layers 16 and 18 could themselves be composed of layers.

Adhesive strip 32 (Figure 2) is secured to the underside 26 of the bag 12 to provide a means of adhesively securing the diaper 10 to an item of wearing apparel. Preferably, a layer of facing material 34 (Figure 2) overlies the entire upper surface and sides 24 of bag 12 to improve the comfort of the diaper 10. Suitable adhesive strips and facing materials are generally well known to those having ordinary skill in the art and in the preferred embodiment the facing is a thermally bonded polypropylene manufactured by Scott.

In use, the adhesive strip 32 adhesively secures the diaper 10 to the wearer's clothing. Preferably, the opening 14 is positioned proximate the perineal area and more specifically aligned with the urethra outlet. When the wearer voids, the urine passes through the opening 14 to the wicking material 16, which rapidly distributes the urine over a large portion of the absorbent material 18. When the urine is so distributed, the absorbent material 18 is capable of absorbing a relatively large flow rate through opening 14. This reduces or even prevents overflow possibilities.

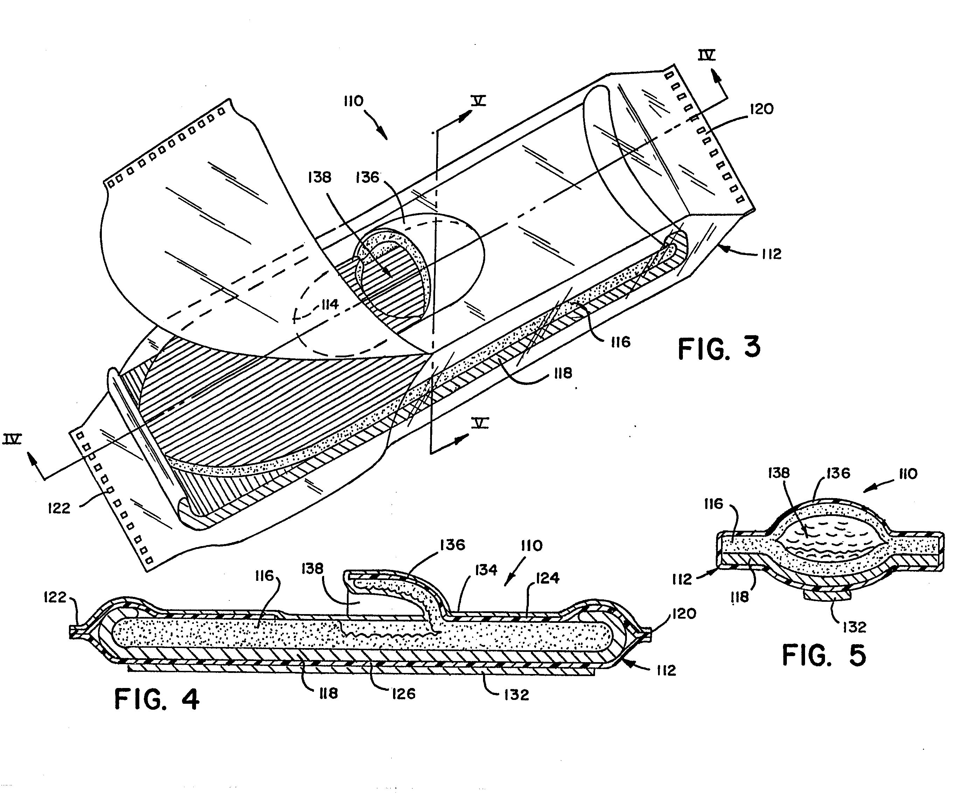

An alternative embodiment 110 of the diaper is illustrated in Figures 3 to 5 and intended for use by males. As in the previously described embodiment, the diaper 110 generally comprises a barrier bag 112, defining an opening 114, and wicking and absorbing layers 116 and 118, respectively, located within the bag. The bag 112 is of tubular stock heat or adhesively sealed at opposite ends 120 and 122 and includes a pouch or flap 136 extending over approximately half of the opening 114. In the preferred embodiment, the flap 136 is integral with the bag 112; however, the flap may comprise a separate piece secured to the bag. The wicking layer 116 is split along the plane of the layer in the area directly under the pouch 136 to define a pocket 138 under the pouch 136. Therefore, the wicking material 116 envelops the pocket 138, as illustrated in Figure 5. Preferably, suitable facing material 134 overlies the upper surface and sides 124 of the bag 112 and extends into and lines the pocket 138 to improve the comfort of the diaper. Adhesive strip 132 is secured to underside 126 so that the diaper may be adhesively secured to an article of clothing. The materials and dimensions of diaper 110 are substantially identical to those of diaper 10.

In use, the wearer's penis is positioned in the pocket 138 to ensure that any urine discharged therefrom is directed through the opening 114 and into the wicking layer 116 to be distributed over the superabsorbent 118. Thus, the diaper 110 provides even further improved liquid reception and absorption capabilities over the unisex diaper 10.

## CLAIMS

1.    An incontinence product (10; 110) comprising:

a waterproof barrier (12; 112) formed into a
flattened, substantially closed bag including upper
and lower surfaces, the upper surface defining an
aperture (14; 114) to be located proximate the perineal
area of a wearer;

a wicking material (16; 116) within said bag
proximate said opening; and

an absorbent material (18; 118) proximate said
wicking material, whereby when the wearer voids, urine
passes through said bag aperture and is rapidly transported by said wicking material to said absorbent
material.

2.    An incontinence product according to Claim
1 in which the absorbent material (18; 118) comprises
a superabsorbent material.

3.    An incontinence product according to Claim 1
or Claim 2 which includes a facing material (34; 134)
overlying the upper surface of the bag (12; 112) to
improve wearer comfort.

4.    An incontinence product according to any of
Claims 1 to 3 which includes adhesive means (32; 132)
secured to the lower surface of the bag (12; 112) for
adhesively securing the product to  an item of wearing
apparel.

5. An incontinence product (110) for males comprising:

a flattened bag (112) fabricated of a liquid-impervious material, the bag including first and second opposite surfaces, one of the said surfaces defining an opening (114) positioned to be located proximate the perineal area of a wearer when the product is worn; and

filler material means (116, 118) within the bag for receiving urine, at least one of the bag (112) and the filler material means (116) including pouch means ·(136 or 138) adjacent said opening for receiving the wearer's penis to ensure that voided urine is directed through said opening into said filler material means.

6. A male incontinence product according to Claim 5 in which the pouch means (136) comprises a flap extending from the bag (112) over a portion of said opening.

7. A male incontinence product according to Claim 6 in which a portion of the filler material means (116) lies adjacent said flap to at least partially line said pouch means to improve the comfort of said pouch means.

8. A male incontinence product according to any of Claims 5 to 7 which includes a facing material (134) overlying said one surface and said pouch means to improve the comfort of the product.

9.    A male incontinence product according to
Claim 8 in which said facing material (134) lines
the pocket.

10.  A male incontinence product according to any
of Claims 5 to 9 in which the filler material means
comprises an absorbent material (116) and a wicking
material (118) positioned between the absorbent
material and the opening (114).

11.  A male incontinence product according to any
of Claims 5 to 10 which includes adhesive means (132)
secured to the other of said bag surfaces for adhesively securing said product to an item of wearing
apparel.

12.  An incontinence product (110) for males
comprising:
a liquid-impervious flattened sack (112) having
upper and lower surfaces, said upper surface defining
an opening (114) into the sack interior, said sack
being substantially closed except for said opening,
said sack including pouch means (136) extending over
a portion of said opening to receive and position the
wearer's penis, whereby the urethra is directed into
said opening;
a wicking material (116) within said sack proximate
said opening to accept and transport urine at a
relatively rapid rate; and
an absorbent material (118) within said bag
proximate said wicking material to absorb the transported urine; said wicking material (116) being separated proximate said pouch means to line said pouch means to further improve wicking and comfort.

13. An incontinence product according to Claim 12 which includes a facing material (134) overlying said upper surface and lining said pouch means (136) to further improve comfort.

FIG. 1

FIG. 2

FIG. 3

FIG. 4

FIG. 5

0140478